# EUROPEAN PATENT SPECIFICATION

(11) **EP 0 497 911 B1**
(45) Date of publication and mention of the grant of the patent: **24.05.2000**
(21) Application number: 90917622.4
(22) Date of filing: 31.10.1990
(51) Int. Cl.: C12N 9/08, C12N 15/00, A61K 38/44

(54) **RECOMBINANT PRODUCTION OF LACTOPEROXIDASE**
REKOMBINANTE HERSTELLUNG VON LAKTOPEROXIDASE
PRODUCTION RECOMBINEE DE LACTOPEROXYDASE

(30) Priority: 03.11.1989 US 431634
(43) Date of publication of application: 12.08.1992
(73) Proprietor: INCYTE PHARMACEUTICALS, INC., Palo Alto , CA 94304 (US)
(72) Inventor: SEILHAMER, Jeffrey, J., Milpitas, CA 95035 (US); DULL, Thomas, J., San Francisco, CA 94122 (US)
(74) Representative: Goldin, Douglas Michael
(86) International application number: US9006317
(87) International publication number: WO9106639

(56) References cited:
- WO-A-89/04608
- FR-A- 2 613 725
- GB-A- 2 162 063
- US-A- 4 576 817
- Pascal On-line database pascal abstract no. 90-0058008
- PROCEEDINGS OF THE NATIONAL ACADEMY OF SCIENCES OF USA vol. 84, August 1987, WASHINGTON US pages 5555 - 5559 KIMURA, S., ET AL. 'Human thyroid peroxidase: Complete cDNA and protein sequence, chromosome mapping, and identification of two alternately spliced mRNAs'
- FEBS LETTERS vol. 250, no. 2, July 1989, AMSTERDAM NL pages 377 - 380 KIMURA, S., ET AL. 'cDNA-directed expression of human thyroid peroxidase'
- DNA AND CELL BIOLOGY vol. 9, no. 7, September 1990, pages 499 - 509 DULL, T.J., ET AL. 'Molecular cloning of cDNAs encoding bovine and human lactoperoxidase'
- Biochemical and Biophysical Resarch Communications, Vol. 133, No. 2, issued 17 December 1985, H.M. GOFF et al., "High Resolution Proton Nuclear Magnetic Resonance Unreadable Text pages 794-799, see entire document.
- Biochemistry Journal, Vol. 259, issued 1989, B. LANGBAKK et al., "Lactoperoxidase from Human Colostrum", pages 627-631, see entire document.
- Biochemical and Biophysical Research Communications, Vol. 160, No. 2, issued 28 April 1989, K.S. BOOTH et al., "Bovine Myeloperoxidase and Lactoperoxidase Each Contain a High Affinity Site for Calcium", pages 897-902, see entire document.
- ACTA Chemica, Grandinavica, Vol. 23, No. 1, issued 1969, A. CARISTROM, "Physical and Compositional Investigations of the Subfractions of Lacteperoxidase", pages 185-202, see entire document.
- FEBS Letters, Vol. 127, No. 2, issued May 1981, G. SIEVERS, "Structure of Milk Lactoperoxidase", pages 253-256, see entire document.
- Immunol. Ser., Vol. 27, issued 1983, K-G. PAUL et al., "The Chemical Structure of Lactoperoxidase", pages 15-29, see entire document.
- FEBS Letters, Vol. 174, No. 2, issued September 1984, B. LANGBAKK et al., "Demonstration and Partial Purification of Lactoperoxidase from Human Colostrum", pages 300-303, see entire document.
- Proceedings National Academy of Sciences, Vol. 78, No. 11, issued November 1981, S.V. SUGGS et al., "Use of Synthetic Oligonucleotides as hybridization Probes: Isolation of Cloned cDNA Sequences for human B2-microglobin", pages 6613-6616, see entire document.

## Description

### Technical Field

The invention relates to the cloning and expression of the gene encoding mammalian lactoperoxidase. The DNAs encoding the bovine and human forms of this protein have been recovered and sequenced.

### Background Art

The presence in milk and in associated secretion glands of an enzyme having peroxidase activity has been known for decades. Carlstrom, A., Acta Chem Scand (1969) 23:185-202, summarized the state of knowledge at that time concerning the heterogeneity of peroxidases in these sources. It was established that the peroxidase activity seemed to reside in a number of interconvertible protein or glycoprotein fractions. Subsequent investigators, for example Sievers, G., FEBS Letters (1981) 127:253-256, described the bovine protein as a glycosylated, single-chain protein having leucine at the N-terminus, and determined the amino acid composition. The protein was shown to contain variable levels of glycosylation and to have a molecular weight of roughly 78 kd. The protein contains a heme group (protoheme IV) at its active site. Further studies of the heterogeneous forms of the bovine protein were published by Paul, K.-G., et al., Immunol Ser (1983) 27:15-29. Langbakk, B., et al., FEBS Lett (1984) 174:300-303, showed that lactoperoxidase was present in human colostrum and disclosed a partial purification procedure for this enzyme. These authors reported that the enzyme isolated from human colostrum had stability, chromatographic and immunoreactive properties similar to that of the lactoperoxidase isolated from bovine milk.

The document, Pascal Mailliart, PhD Thesis, University of Paris VII, "Contribution a la valorisation des proteines du lactoserum: de la purification au sequencage", publicly defended on 26/1/89, discloses, inter alia, N-terminal sequences of bovine lactoperoxydase proteins.

Lactoperoxidase is a highly useful protein, as it has antimicrobial activity which permits its use as a fungicide, viricide, protozoacide and bacteriocide both in products which need preservatives or therapeutically. In addition, because of its peroxidase activity, it can be used as a labeling and/or linking reagent in the conduct of various diagnostic and analytical assays. The production of this protein in recombinant form provides a reliable and reproducible source of this important protein.

### Disclosure of the Invention

The invention provides DNA sequences encoding mammalian lactoperoxidase enzymes and recombinant expression systems for the production of the recombinant lactoperoxidase proteins. The protein produced has wide industrial, therapeutic, and diagnostic applications in humans and in animals.

Thus the invention is directed to isolated DNA sequences encoding these proteins, to expression systems capable of producing these proteins, and to recombinant host cells transformed with these expression systems, and to methods of production using these systems and cells. In addition, the invention is directed to pharmaceutical compositions containing the recombinant lactoperoxidase proteins of the invention as well as formulations suitable for diagnostic and industrial use. The invention is also directed to methods of preserving organic compositions using lactoperoxidase and to methods of treating various conditions susceptible to this enzyme.

### Brief Description of the Drawings

Figure 1 shows the complete DNA sequence encoding bovine lactoperoxidase preproenzyme and the deduced amino acid sequence.

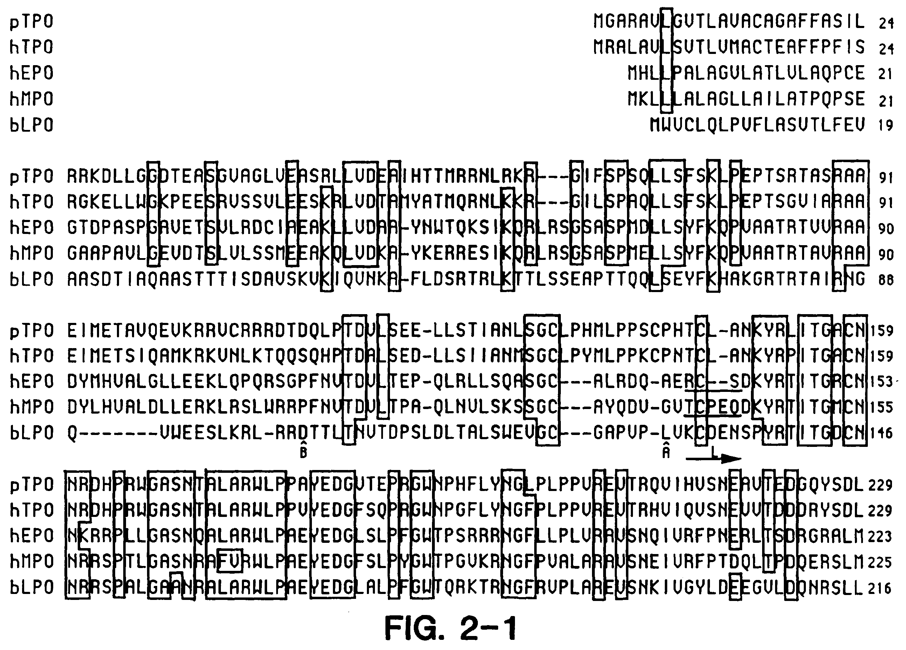

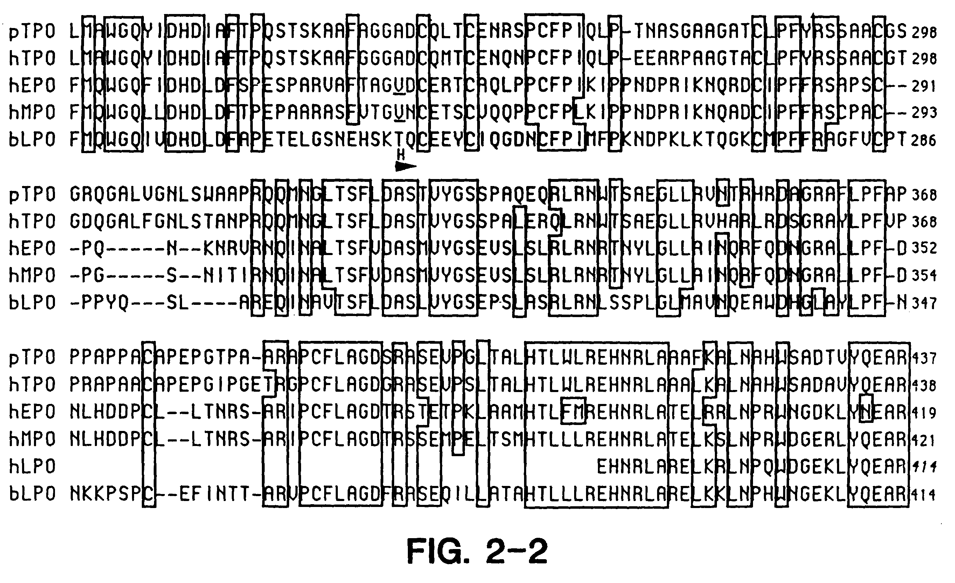

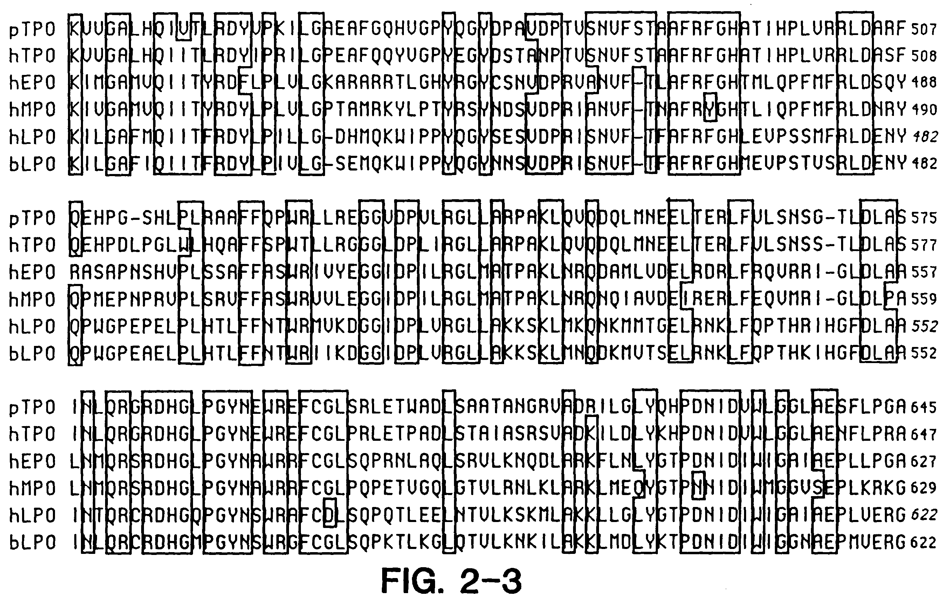

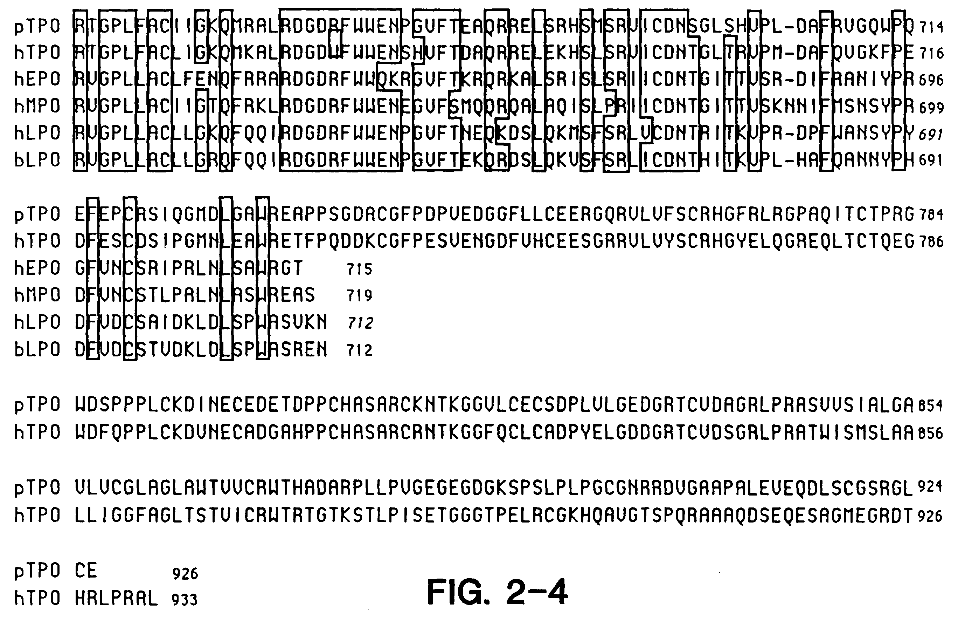

Figure 2 shows a comparison of the amino acid sequences of a number of mammalian peroxidases.
Figure 3 shows the sequence of a human cDNA clone.

### Modes of Carrying Out the Invention

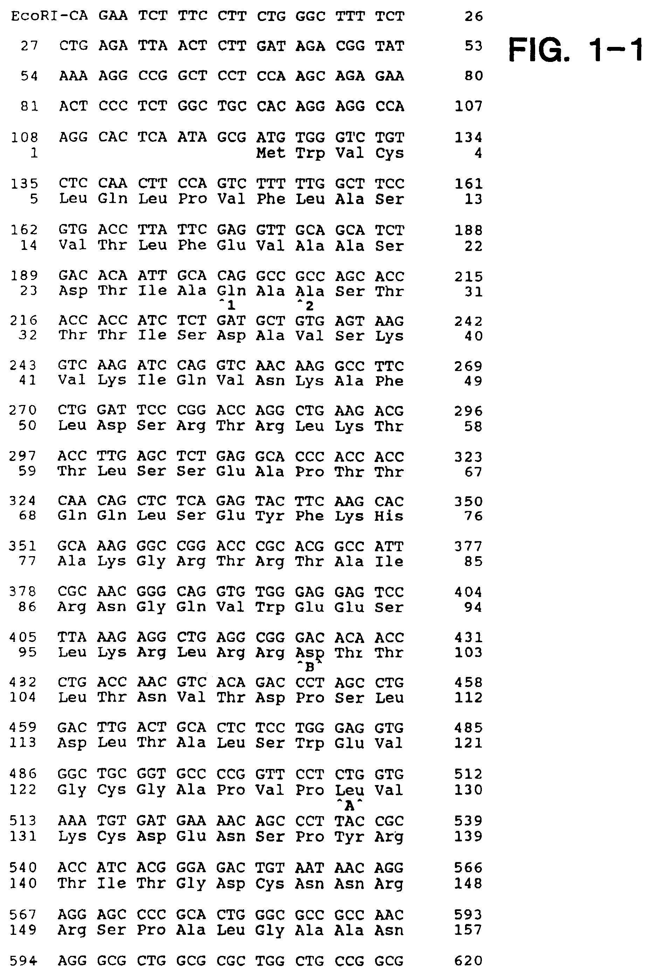

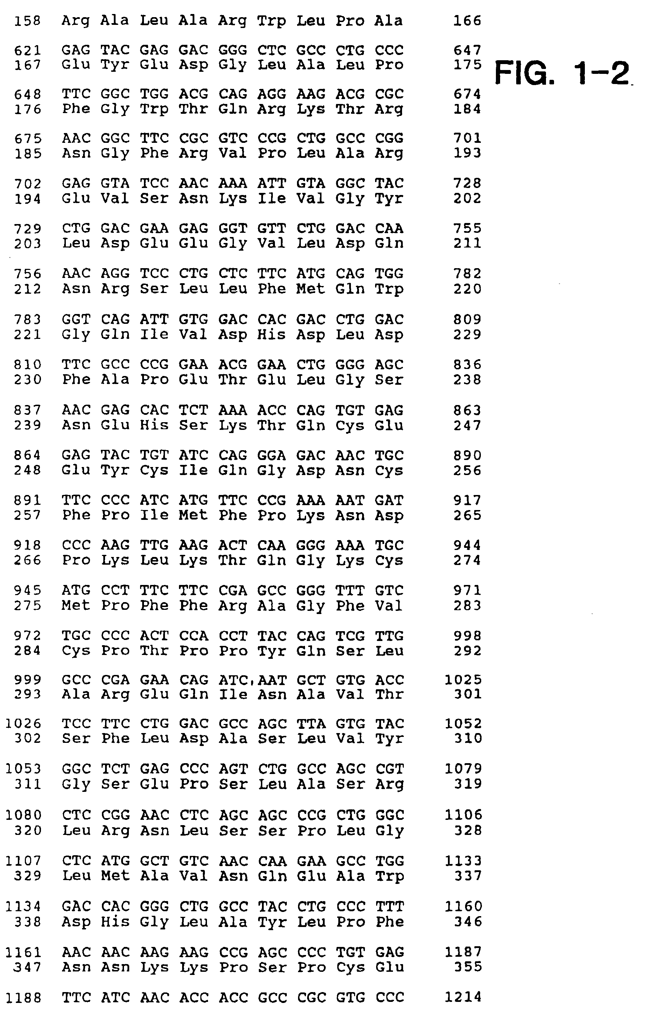

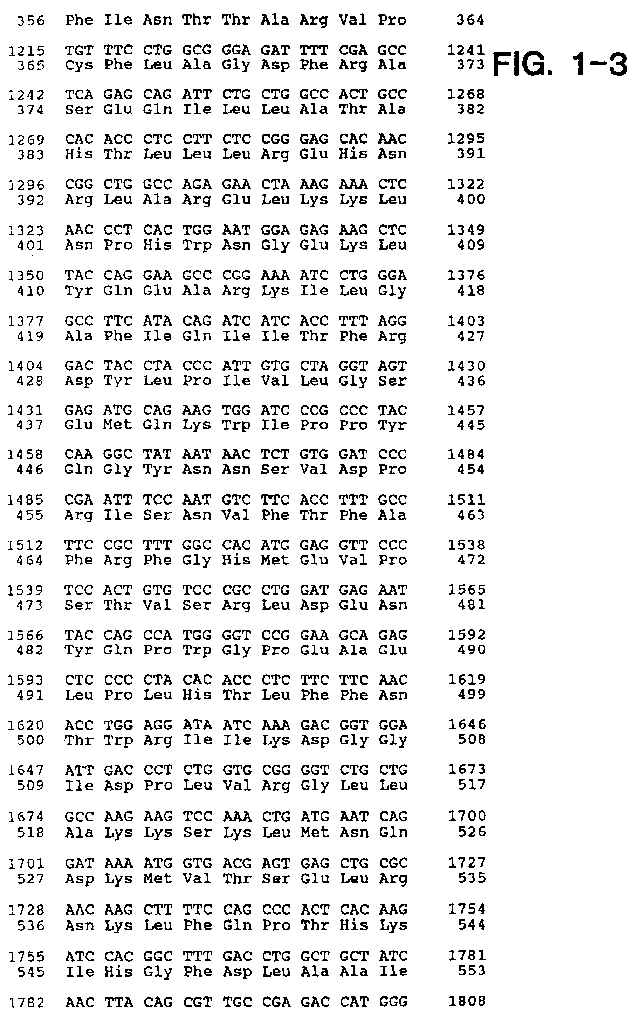

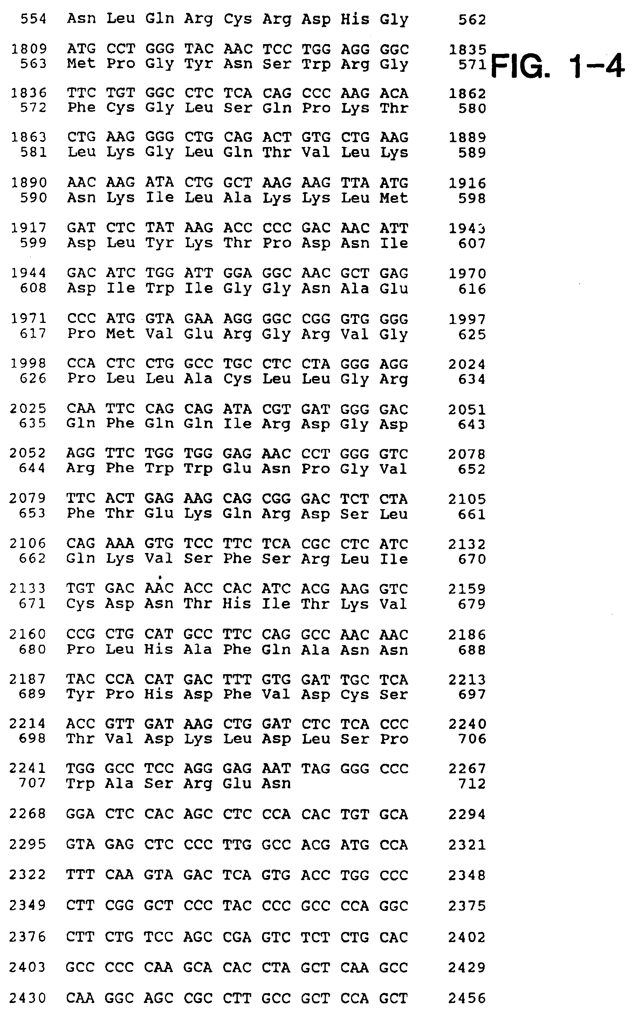

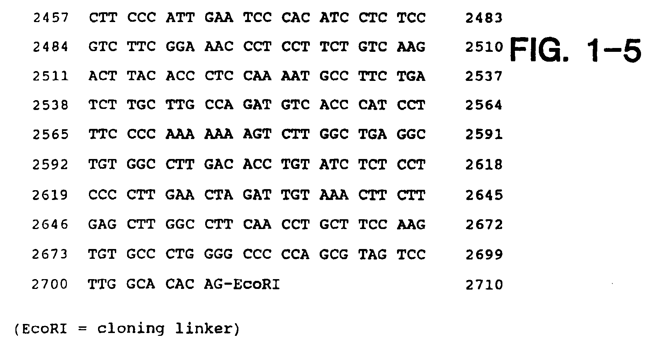

As used herein, "mammalian" lactoperoxidase refers to a protein having peroxidase activity, as assayed by standard enzymological tests, and which protein is present in the secretions of milk-secreting glands and in the glands themselves. Such conventional tests for peroxidase activity are commercially available and described in standard references such as Methods in Enzymology. Illustrative of such proteins are the bovine protein having the amino acid sequence shown in Figure 1 and the related human sequence.

"Operably linked" refers to a juxtaposition wherein the components are configured so as perform their usual functions; thus, control sequences or promoters operably linked to a coding sequence are capable of effecting the expression of the coding sequence.

"Control sequence" refers to a DNA sequence or sequences which are capable, when properly ligated to a desired coding sequence, of effecting its expression in hosts compatible with such sequence. These sequences include at least promoters, both in procaryotic and eucaryotic hosts, and, optionally, transcription termination signals. Additional sequences which may be required or helpful in effecting expression may also be identified and incorporated. As used herein, "control sequences" refers to whatever DNA sequence may be required to effect expression of the operably linked coding sequence in the particular host used.

"Cells" or "cell cultures" or "host cells" are often used interchangeably, as will be clear from the context. These terms include the immediate subject cell, and, of course, the progeny thereof. It is understood that not all progeny are exactly identical to the parental cell due to chance mutation or differences in environment, but such altered progeny are included in these terms as long as they retain the characteristics relevant to those confirmed on an originally transformed cell. In the present case, for example, the included progeny would be those which retain an expression system capable of effecting the production of the desired lactoperoxidase.

The DNA sequences encoding the illustrative bovine and human lactoperoxidases of the invention were obtained from cDNA libraries obtained from bovine and human milk glands. In the case of these illustrative proteins, bovine lactoperoxidase (LPO) was purified from commercial preparations of lactoperoxidase, and internal amino acid sequence information was obtained from CNBr fragments of the purified material. Probes designed from these determined sequences were used to screen a cDNA library from bovine milk gland and three clones were obtained which encoded the bovine LPO sequence shown in Figure 1. The sequence contains 712 amino acids, including the signal sequence and pro sequence. Two putative signal peptidase cleavage sites (1 and 2) and two possible alternative termini of the cleaved propeptide (A and B) are indicated on the figure. Thus the mature protein may begin at the Asp at 101 or the Leu at 129.

Genomic DNA was also prepared from a bovine library and the retrieved bLPO sequence used to perform Southern blots on SDS gels of the genomic bank. The results of these Southern blots indicated that multiple genes may encode bovine lactoperoxidase proteins; however, the multiple bands found may also be attributable to related peroxidases.

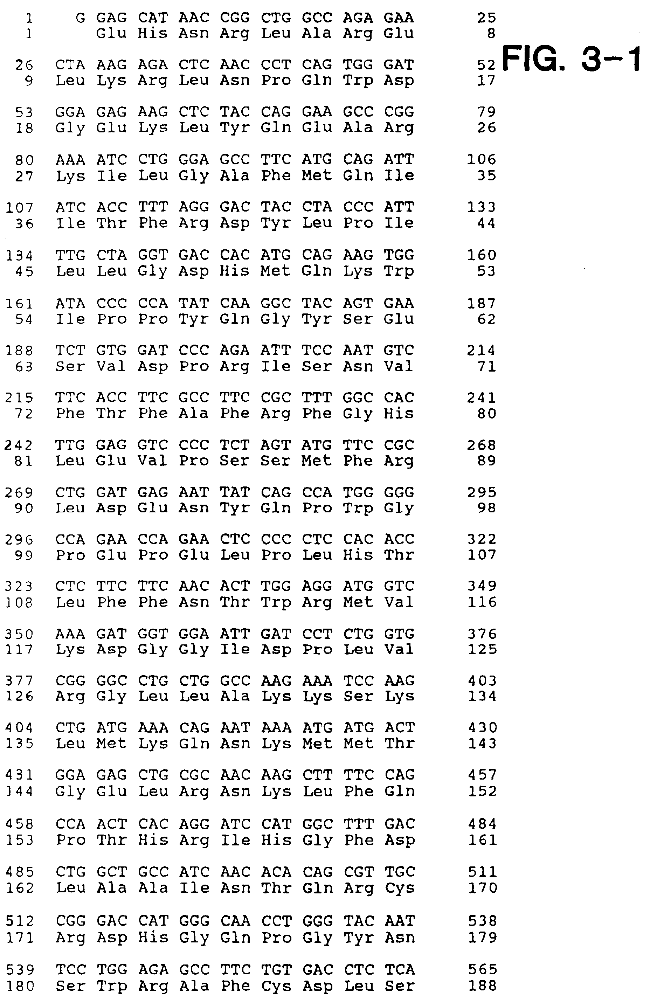

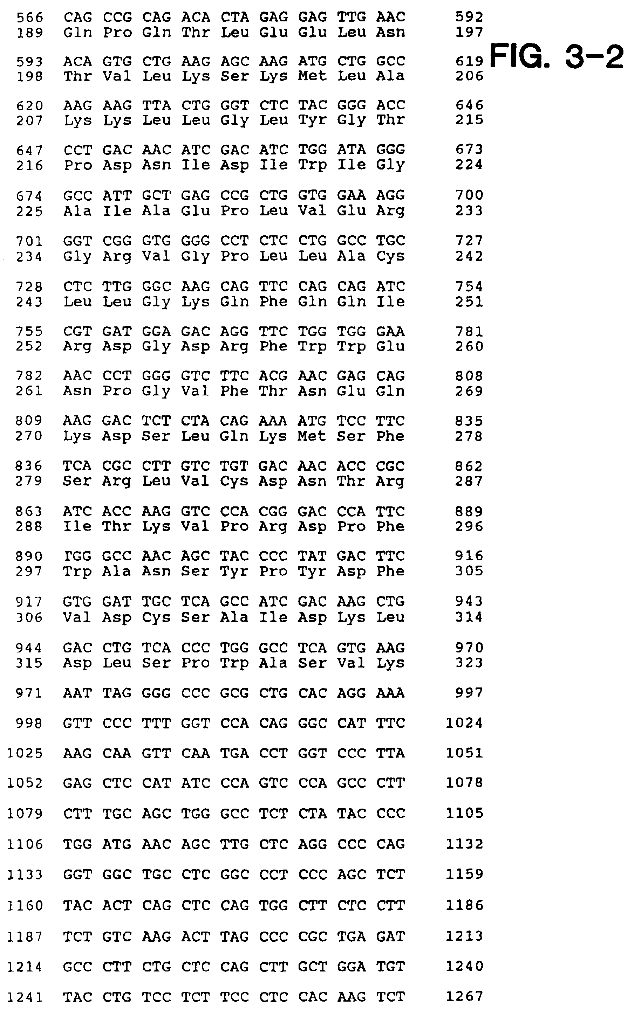

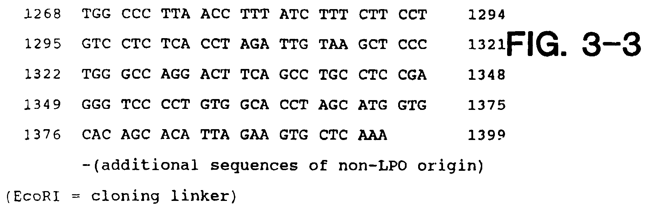

The bovine clone was also used to screen a human mammary tissue cDNA library, and a clone encoding the carboxy-terminal 324 residues of human cDNA for LPO was isolated. The human sequence shown in Figure 3 was found to be highly homologous to the bovine protein.

The illustrated bovine and human sequences permit the retrieval of the corresponding LPO-encoding genes from cDNA or genomic libraries prepared from other mammalian species. As high homology is expected between these species, stringent conditions can be used, thus eliminating false positives. Examples of stringent conditions include hybridization at 4 x SSC at 65°C followed by one hour washing in 0.1 x SSC at 65°C, or hybridization in 50% formamide, 4 x SSC, at 42°C followed by washing in 0.1 x SSC at 65°C for an hour.

Thus, as the bovine sequence is available, a cDNA or genomic DNA encoding lactoperoxidases present in other mammalian sources can also be recovered either from genomic or cDNA banks using this sequence as a probe. Hybridization under high stringency with the DNA of Figure 1 is possible due to the homology of the lactoperoxidases in mammals. In addition, lactoperoxidases may be recovered from cDNA libraries prepared from mammary glands by hybridization under stringent conditions to DNA probes which simply encode the amino acid sequence shown in Figure 1--i.e., may be degenerate, due to the redundancy of the code, with the cDNA sequence there shown.

### Utility and Administration

The lactoperoxidases of the invention are useful, in one aspect, as antimicrobial agents both in therapeutic contexts and to preserve a variety of perishable goods. The lactoperoxidases of the invention are capable of killing fungi, viruses, protozoa and bacteria, and are effective agents against infectious diseases as well as disorders of the immune system such as malignancies, autoimmune disease, and transplant rejections.

For therapeutic purposes, the lactoperoxidase of the invention is formulated according to standard procedures for the formulation of proteinaceous active ingredients, a review of which is found in Remington's Pharmaceutical Sciences, Mack Publishing Company, Easton, Pennsylvania, latest edition. The therapeutic compositions are administered, preferably, systemically, most commonly by injection, either intravenously, intramuscularly, subcutaneously, or intraperitoneally. Formulations for injection are generally aqueous solutions or suspensions in physiologically compatible buffers such as Hank's solution or Ringer's solution. In addition, the protein may be formulated as a solid, for example, by lyophilization, and then reconstituted in suitable liquid for administration to the subject.

In addition, systemic administration may be achieved by transdermal or transmucosal administration using a membrane penetrant such as bile salts or fusidic acids and their analogs. Suitable pharmaceutically acceptable nontoxic detergents may also be used. The administration across membranes can be effected at vulnerable locations, especially, such as through nasal sprays or suppositories.

Although it is more challenging to do so, the proteins of the invention may also be administered orally when properly formulated to protect them from digestion.

In addition, it has lately been possible to administer proteins through in situ production thereof as a result of expression of a gene transformed into the subject. Thus, an alternate route of administration is through gene therapy to obtain a transgenic subject.

In addition, the lactoperoxidases of the invention may be administered locally to counteract, for example, localized infection or malignancy. Such local administration may be topical in the form of ointments, creams, salves, or gels, or may be by a transdermal patch containing a limited amount of drug, or by local subcutaneous injection.

The dosage and route of administration are highly dependent on the nature of the subject, the nature of the conditions being treated, and the judgment of the physician or veterinarian. However, suitable dosage ranges for treatment of infectious diseases are of the order of 0.01-100 mg/kg/day.

In addition to use as therapeutic agents, the proteins of the invention can be used to stabilize perishable goods such as foods, beverages, cosmetics, and the like. For use in these applications, the protein can be added to the material in an amount of 0.01-5%, typically.

Finally, the lactoperoxidases of the invention may also be used as labeling reagents for immunoassays or other specific binding assays. In these applications, the enzyme is conjugated to a component of the test system designed to identify the analyte characteristic of, for example, a disease condition. Applications in ELISA assays, for example, are analogous to the use of horseradish peroxidase in these systems.

### Retrieval of the Bovine Lactoperoxidase Gene

A cDNA library is prepared from milk gland cells of bovine teats using standard procedures, as follows: A preparation of mRNA is obtained from these cells using standard extraction and oligo-dT chromatographic techniques. The cDNA library is then prepared, for example, using the lambda-gt10/lambda-gt11 vectors of Huynh, V.T., et al., DNA Cloning Techniques: A Practical Approach (IRL Press, Oxford, 1984). In the present work, the commercially available lambda-ZAP-II vector from Statagene, San Diego, CA, was employed using the manufacturer's instructions.

This library is then screened with probes designed to correspond to the sequence obtained from isolated bovine lactoperoxidase protein. The bovine lactoperoxidase is purified from commercially available proteins using an HPLC column and recovering a single peak. The recovered protein is then hydrolyzed using CNBr, and the fragments are sequenced. A set of oligonucleotide probes is then designed on the basis of two sequences obtained in the full-length protein.

The library from bovine teats was probed using the two kinased oligomers, and three clones were recovered which hybridized to both. The screening was done under standard, moderately stringent conditions. All three retrieved clones contained portions of the open reading frame shown in Figure 1 encoding 712 amino acids, and one clone contained the complete sequence shown in Figure 1. This clone was designated pIDNbLPO-1 and was deposited at the American Type Culture Collection, Rockville, MD, on 2 November 1989 under the conditions of the Budapest Treaty. The mature protein begins at the aspartic acid residue shown in position 101. Since lactoperoxidase is a secreted protein, the upstream portions contain both a signal and a pro-LPO sequence. A human clone, pIDNhLPO-3, with the sequence shown in Figure 3, was also deposited 2 November 1989 at the ATCC under the conditions of the Budapest Treaty.

The resulting clone is then ligated into suitable expression systems and transformed into host cells for production of the protein. The LPO can be produced as an intracellular protein by removal of the DNA sequence encoding the leader and pro sequence and inserting at ATG start codon upstream of the N-terminal aspartic acid residue. Alternatively, the heterologous or homologous signal sequences can be used to effect the secretion of the protein.

Construction of expression systems operable in a spectrum of host cells is well within ordinary skill. The retrieved cDNA or synthetic or partially synthetic DNA encoding the desired protein is ligated to control sequences appropriate to the intended host. The coding sequence ligated to appropriate controls can be included on a self-replicating vector, as is ordinarily the case when yeast or procaryotic hosts are used, or may be designed to integrate into the host cells' chromosome, as is often the case for mammalian cell hosts.

For procaryotic expression, suitable promoters include the regulatable trp promoter, the regulatable lac promoter, or the commercially available hybrid of these systems, variously designated the trc or tac promoter. Signal sequences operable in bacteria are available from the penicillinase gene, which can also be used with its own promoter. Typical replicating vectors suitable for transformation of E. coli, for example, include the pBR322 derived vectors and vectors related to the pUC series. However, other bacterial hosts with suitable modifications of the control systems in vectors can also be used, including, for example, Bacillus subtillus various pseudo-monads, and related host strains. However, expression in E. coli is generally the most convenient.

Yeasts are also commonly used as eukaryotic hosts, and a number of promoters, especially those which are indigenous as promoters for synthesis of the glycolytic enzymes, such as the 3-phosphoglycerate kinase (PGK) promoter, the enolase promoter, or the promoter associated with the Leu2 gene can be used. Signal sequences which are operable in yeast include those derived from the alpha-factor gene. Yeast vectors commonly include a replication site, such as the two-micron replicon.

Suitable mammalian expression systems generally comprise viral promoters such as the SV40, polyoma, adenovirus, bovine papilloma virus (BPV), or avian sarcoma virus promoters. However, also useful in mammalian systems are indigenous promoters such as the regulatable metallothionein promoter. Additional controls are generally desirable in mammalian systems including terminating sequences for transcription and various enhancers. Signal sequences applicable to mammalian systems include those associated with normally-secreted mammalian proteins such as the various growth hormones and insulin.

In addition, although less convenient, plant cells can also be used as hosts using appropriate controls such as nopaline synthetase promoter. A commonly used system for production of recombinant proteins includes the bacculovirus/insect host system.

Additional features may be included in the expression systems such as, for example, in mammalian systems, amplification by cotransformation with an amplifiable gene such as that encoding dihydrofolic reductase (DHFR).

Depending on the nature of the expression system chosen, the desired lactoperoxidase is recovered and purified from the recombinantly transformed host. The host cell is cultured under conditions favorable for the expression of the lactoperoxidase gene--e.g., in prokaryotic systems, if expression is under control of the trp promoter, diminished concentrations of tryptophan or the presence of a tryptophan inhibitor, such as indoleacetic acid; in mammalian systems where the lactoperoxidase gene may be under control of the metallothionein system, in the presence of metal ions capable of inducing this promoter.

If the lactoperoxidase is produced as intracellular protein, the cells are lysed, and the lactoperoxidase separated from the cellular proteins. In general, it is more convenient to produce the lactoperoxidase as a secreted protein and to recover it from the medium as the levels of contaminating proteins are lower.

Standard purification procedures are employed such as ion exchange chromatography, affinity chromatography, differential centrifugation, ammonium sulfate precipitation, gel filtration and the like.

## Claims (Claims for the following Contracting State(s): AT, BE, CH, DE, DK, FR, GB, LI, LU, IT, NL, SE)

1. An isolated DNA sequence which comprises the nucleotide sequence of Figure 1 or 3, or a sequence which is complementary to it.

2. A DNA sequence according to claim 1 which encodes a protein whose amino acid sequence is given in Figure 1 (bovine) or 3 (human) that has peroxidase activity.

3. An expression system which, when contained in a recombinant host cell, can express a DNA sequence encoding a protein with lactoperoxidase activity, wherein the expression system comprises: (a) a DNA sequence defined in claim 1 or 2 operably linked to (b) control systems compatible with the host cell to enable expression of the DNA sequence.

4. A recombinant host cell which contains an expression system according to claim 3 or a recombinantly transformed host cell transformed with an expression system according to claim 3.

5. A host cell according to claim 4 which is a mammalian cell.

6. A method of producing a protein with lactoperoxidase activity comprising culturing recombinant host cells according to claim 4 or 5 under conditions suitable to effect expression of DNA encoding the protein; and
recovering the protein produced.

7. A method of preserving a perishable product, the method comprising adding to the product an amount of recombinant mammalian lactoperoxidase encoded by DNA according to claim 1 or 2 effective to preserve the product.

8. A method of crosslinking ligands to proteins, the method comprising conjugating recombinant mammalian lactoperoxidase encoded by DNA according to claim 1 or 2 to the protein and the ligand.

9. A method of labelling an immunoassay reagent, the method comprising conjugating the reagent with recombinant mammalian lactoperoxidase encoded by DNA according to claim 1 or 2.

10. A method according to any of claims 7 to 9 wherein the lactoperoxidase is produced by a method according to claim 6.

11. An isolated DNA sequence encoding bovine lactoperoxidose, the DNA comprising the sequence of nucleotides 123 to 2258 of Figure 1.

12. An isolated DNA sequence encoding the carboxy terminus of human lactoperoxidase, the DNA comprising the sequence of nucleotides 2 to 970 of Figure 3.

## Claims (Claims for the following Contracting State(s): ES)

1. A recombinant host cell which contains an expression system or a recombinantly transformed host transformed with an expression system, the expression system comprising a DNA sequence which comprises the nucleotide sequence of Figure 1 or 3, or a sequence which is complementary to it, operably linked to control systems compatible with the host to enable expression of the DNA sequence.

2. A host cell according to claim 1 which expresses a protein whose amino acid sequence is given in Figure 1 (bovine) or 3 (human) that has peroxidase activity.

3. A host cell according to claim 1 or 2 which is a mammalian cell.

4. A method of producing a protein with lactoperoxidase activity comprising culturing recombinant host cells as defined in any preceding claim under conditions suitable to effect expression of DNA encoding the protein; and
recovering the protein produced.

5. A method of preserving a perishable product, the method comprising adding to the product an amount of recombinant mammalian lactoperoxidase encoded by DNA as defined in claim 1 effective to preserve the product.

6. A method of crosslinking ligands to proteins, the method comprising conjugating recombinant mammalian lactoperoxidase encoded by DNA as defined in claim 1 to the protein and the ligand.

7. A method of labelling an immunoassay reagent, the method comprising conjugating the reagent with recombinant mammalian lactoperoxidase encoded by DNA as defined in claim 1.

8. A method according to any of claims 5 to 7 wherein the lactoperoxidase is produced by a method according to claim 4.

## Patentansprüche (Patentansprüche für folgende(n) Vertragsstaat(en): AT, BE, CH, DE, DK, FR, GB, LI, LU, IT, NL, SE)

1. Isolierte DNA-Sequenz, die die Nucleotidsequenz von Figur 1 oder 3 umfasst, oder eine Sequenz, die dazu komplementär ist.

2. DNA-Sequenz nach Anspruch 1, die ein Protein mit Peroxidaseaktivität codiert, dessen Aminosäuresequenz in Figur 1 (Rind) oder 3 (Mensch) gezeigt ist.

3. Expressionssystem, das, wenn es in einer rekombinanten Wirtszelle enthalten ist, eine DNA-Sequenz exprimieren kann, die ein Protein mit Lactoperoxidaseaktivität codiert, wobei das Expressionssystem umfasst: (a) eine DNA-Sequenz gemäß der Definition in Anspruch 1 oder 2 funktionell verknüpft mit (b) Kontrollsystemen, die mit der Wirtszelle kompatibel sind, so daß die Expression der DNA-Sequenz möglich ist.

4. Rekombinante Wirtszelle, die ein Expressionssystem nach Anspruch 3 oder eine rekombinant transformierte Wirtszelle enthält, die mit einem Expressionssystem nach Anspruch 3 transformiert ist.

5. Wirtszelle nach Anspruch 4, die eine Säugerzelle ist.

6. Verfahren zur Produktion eines Proteins mit Lactoperoxidaseaktivität, umfassend das Züchten rekombinanter Wirtszellen nach Anspruch 4 oder 5 unter Bedingungen, die geeignet sind, eine Expression von das Protein codierender DNA zu bewirken, und
Gewinnung des produzierten Proteins.

7. Verfahren zur Konservierung eines verderblichen Produkts, wobei das Verfahren die Zugabe rekombinanter Säuger-Lactoperoxidase, die durch DNA nach Anspruch 1 oder 2 codiert wird, zu dem Produkt in einer Menge umfasst, die die Konservierung des Produkts bewirkt.

8. Verfahren zur Vernetzung von Liganden mit Proteinen, wobei das Verfahren die Konjugation von rekombinanter Säuger-Lactoperoxidase, die von der DNA nach Anspruch 1 oder 2 codiert wird, mit dem Protein und dem Liganden umfasst.

9. Verfahren zur Markierung eines Immuntest-Reagenzes, wobei das Verfahren die Konjugation des Reagenzes mit rekombinanter Säuger-Lactoperoxidase, die von DNA nach Anspruch 1 oder 2 codiert wird, umfasst.

10. Verfahren nach einem der Ansprüche 7 bis 9, wobei die Lactoperoxidase gemäß einem Verfahren nach Anspruch 6 produziert wird.

11. Isolierte DNA-Sequenz, die Rinder-Lactoperoxidase codiert, wobei die DNA die Sequenz der Nucleotide 123 bis 2258 von Figur 1 umfasst.

12. Isolierte DNA-Sequenz, die das carboxyterminale Ende von menschlicher Lactoperoxidase codiert, wobei die DNA die Sequenz der Nucleotide 2 bis 970 von Figur 3 umfasst.

## Patentansprüche (Patentansprüche für folgende(n) Vertragsstaat(en): ES)

1. Rekombinante Wirtszelle, die ein Expressionssystem oder eine rekombinant transformierte Wirtszelle enthält, die mit einem Expressionssystem transformiert ist, wobei das Expressionssystem eine DNA-Sequenz, die die Nucleotidsequenz von Figur 1 oder 3 umfasst, oder eine Sequenz, die dazu komplementär ist, funktionell verknüpft mit Kontrollsystemen, die mit der Wirtszelle kompatibel sind, so daß die Expression der DNA-Sequenz möglich ist, umfaßt.

2. Wirtszelle nach Anspruch 1, die ein Protein mit Peroxidaseaktivität exprimiert, dessen Aminosäuresequenz in Figur 1 (Rind) oder 3 (Mensch) gezeigt ist.

3. Wirtszelle nach Anspruch 1 oder 2, die eine Säugerzelle ist.

4. Verfahren zur Produktion eines Proteins mit Lactoperoxidaseaktivität, umfassend das Züchten rekombinanter Wirtszellen gemäß der Definition in einem der vorangehenden Ansprüche unter Bedingungen, die geeignet sind, eine Expression von das Protein codierender DNA zu bewirken, und
Gewinnung des produzierten Proteins.

5. Verfahren zur Konservierung eines verderblichen Produkts, wobei das Verfahren die Zugabe rekombinanter Säuger-Lactoperoxidase, die durch DNA nach Anspruch 1 codiert wird, zu dem Produkt in einer Menge umfasst, die die Konservierung des Produkts bewirkt.

6. Verfahren zur Vernetzung von Liganden mit Proteinen, wobei das Verfahren die Konjugation von rekombinanter Säuger-Lactoperoxidase, die von der DNA nach Anspruch 1 codiert wird, mit dem Protein und dem Liganden umfasst.

7. Verfahren zur Markierung eines Immuntest-Reagenzes, wobei das Verfahren die Konjugation des Reagenzes mit rekombinanter Säuger-Lactoperoxidase, die von DNA nach Anspruch 1 codiert wird, umfasst.

8. Verfahren nach einem der Ansprüche 5 bis 7, wobei die Lactoperoxidase gemäß einem Verfahren nach Anspruch 4 produziert wird.

## Revendications (Revendications pour l'(les) Etat(s) contractant(s) suivant(s): AT, BE, CH, DE, DK, FR, GB, LI, LU, IT, NL, SE)

1. Séquence d'ADN isolée, qui comprend la séquence nucléotidique de la figure 1 ou de la figure 3, ou une séquence qui lui est complémentaire.

2. Séquence d'ADN selon la revendication 1, qui code une protéine, dont la séquence d'acides aminés est donnée à la figure 1 (bovine) ou à la figure 3 (humaine), qui a une activité peroxydase.

3. Système d'expression, qui, lorsqu'il est contenu dans une cellule hôte recombinante, peut exprimer une séquence d'ADN codant une protéine ayant une activité lactoperoxydase, le système d'expression comprenant : (a) une séquence d'ADN définie dans la revendication 1 ou la revendication 2, liée de façon opérationnelle à (b) des systèmes de contrôle compatibles avec la cellule hôte pour permettre l'expression de la séquence d'ADN.

4. Cellule hôte recombinante qui contient un système d'expression selon la revendication 3 ou cellule hôte transformée par recombinaison selon la revendication 3.

5. Cellule hôte selon la revendication 4, qui est une cellule de mammifère.

6. Méthode de production d'une protéine ayant une activité lactoperoxydase, comprenant la culture de cellules hôtes recombinantes selon la revendication 4 ou la revendication 5, dans des conditions permettant d'effectuer l'expression d'ADN codant la protéine ; et
la récupération de la protéine produite.

7. Méthode de conservation d'un produit périssable, la méthode comprenant l'addition au produit d'une quantité de lactoperoxydase de mammifère recombinante codée par un ADN selon la revendication 1 ou la revendication 2, qui est efficace pour conserver le produit.

8. Méthode de réticulation de ligands à des protéines, la méthode comprenant la conjugaison de lactoperoxydase de mammifère recombinante codée par un ADN selon la revendication 1 ou la revendication 2 à la protéine et au ligand.

9. Méthode de marquage d'un réactif de dosage immunologique, la méthode comprenant la conjugaison du réactif avec une lactoperoxydase de mammifère recombinante codée par un ADN selon la revendication 1 ou la revendication 2.

10. Méthode selon l'une quelconque des revendications 7 à 9, dans laquelle la lactoperoxydase est produite par une méthode selon la revendication 6.

11. Séquence d'ADN isolée codant une lactoperoxydase bovine, l'ADN comprenant la séquence des nucléotides 123 à 2258 de la figure 1.

12. Séquence d'ADN isolée codant l'extrémité carboxy-terminale de lactoperoxydase humaine, l'ADN comprenant la séquence des nucléotides 2 à 970 de la figure 3.

## Revendications (Revendications pour l'(les) Etat(s) contractant(s) suivant(s): ES)

1. Cellule hôte recombinante qui contient un système d'expression ou hôte transformé par recombinaison par un système d'expression, le système d'expression contenant une séquence d'ADN qui comprend la séquence nucléotidique de la figure 1 ou de la figure 3, ou une séquence qui lui est complémentaire, liée de façon opérationnelle à des systèmes de contrôle compatibles avec l'hôte afin de permettre l'expression de la séquence d'ADN.

2. Cellule hôte selon la revendication 1, qui exprime une protéine dont la séquence d'acides aminés est donnée à la figure 1 (bovine) ou la figure 3 (humaine), qui a une activité peroxydase.

3. Cellule hôte selon la revendication 1 ou la revendication 2, qui est une cellule de mammifère.

4. Méthode de production d'une protéine ayant une activité lactoperoxydase, comprenant la culture de cellules hôtes recombinantes telles que définies dans l'une quelconque des revendications précédentes, dans des conditions permettant d'effectuer l'expression d'ADN codant la protéine ;
et
la récupération de la protéine produite.

5. Méthode de conservation d'un produit périssable, la méthode comprenant l'addition au produit d'une quantité de lactoperoxydase de mammifère recombinante codée par un ADN tel que défini dans la revendication 1, qui est efficace pour conserver le produit.

6. Méthode de réticulation de ligands à des protéines, la méthode comprenant la conjugaison de lactoperoxydase de mammifère recombinante codée par un ADN tel que défini dans la revendication 1 à la protéine et au ligand.

7. Méthode de marquage d'un réactif de dosage immunologique, la méthode comprenant la conjugaison du réactif avec une lactoperoxydase de mammifère recombinante codée par un ADN tel que défini dans la revendication 1.

8. Méthode selon l'une quelconque des revendications 5 à 7, dans laquelle la lactoperoxydase est produite par une méthode selon la revendication 4.
